# EUROPEAN PATENT APPLICATION

(11) **EP 4 786 966 A1**
(43) Date of publication of application: **05.08.2026**
(21) Application number: 24871529.4
(22) Date of filing: 08.08.2024
(51) Int. Cl.: G01N 27/12, G01N 27/04

(54) **GAS SENSOR**

(30) Priority: 27.09.2023 JP 2023166127; 26.06.2024 JP 2024102703
(71) Applicant: Niterra Co., Ltd., Nagoya-shi, Aichi 461-0005 (JP)
(72) Inventor: KONDO Tomonori, Nagoya-shi, Aichi 461-0005 (JP); KATO Tomofumi, Nagoya-shi, Aichi 461-0005 (JP); IKAWA Koichi, Nagoya-shi, Aichi 461-0005 (JP); TAKAKURA Masahiro, Nagoya-shi, Aichi 461-0005 (JP)
(74) Representative: Diehl & Partner
(86) International application number: PCT/JP2024/028428
(87) International publication number: WO 2025/069727

(57) **Abstract**

A gas sensor 100 includes a stacked body 11 in which electrode layers 31 and 32 and an insulating layer 24 alternatingly stacked. The stacked body 11 has a sloping surface 11C at a portion at least of its side surface. The sloping surface 11C functions as a detection portion which detects a particular component of a gas under measurement.

## Description

### TECHNICAL FIELD

The present disclosure relates to a gas sensor.

### BACKGROUND ART

Conventionally, as a technique relating to a sensor, the technique described in Patent Literature 1 has been known. Specifically, Patent Literature 1 discloses a sensor in which a first Au layer is formed on a silicon substrate and a second Au layer is formed to be separated from the first Au layer by using Al₂O₃, whereby a nanogap whose size corresponds to the thickness of the Al₂O₃ is formed. When a solution is injected to the nanogap of the sensor, the sensor can detect a particular substance through a change in an electrical property between opposite ends of the nanogap.

### CITATION LIST

### PATENT LITERATURE

Patent Literature 1: JP2006-234799A

### SUMMARY OF INVENTION

### TECHNICAL PROBLEM

It is desired for the configuration disclosed in Patent Literature 1 to improve its sensitivity of detecting the particular substance. However, heretofore, the structure of the electrode layers of the sensor has not been studied for improvement of the sensitivity of detecting the particular substance.

The present disclosure discloses a technique which has been completed on the basis of the above-described circumstances, and one object is to provide a gas sensor which can improve the sensitivity of detection.

### SOLUTION TO PROBLEM

A gas sensor of the present disclosure comprises a stacked body including electrode layers and an insulating layer alternatingly stacked, wherein the stacked body has a sloping surface at a portion at least of its side surface, and the sloping surface functions as a detection portion which detects a particular component of a gas under measurement.

### ADVANTAGEOUS EFFECT OF INVENTION

According to the present disclosure, it becomes possible to provide a gas sensor which can improve the sensitivity of detection.

### BRIEF DESCRIPTION OF DRAWINGS

[FIG. 1] Plan view schematically representing a gas sensor device according to Embodiment 1.
[FIG. 2] View showing the sectional configuration of a portion of a gas sensor.
[FIG. 3] Sectional view showing, on an enlarged scale, a sloping surface and its vicinity of a stacked body.
[FIG. 4] View showing the sectional configuration of a portion of a gas sensor according to Embodiment 2.
[FIG. 5] Plan view schematically representing a gas sensor device according to Embodiment 3.
[FIG. 6] View showing the sectional configuration of a portion of a gas sensor.
[FIG. 7] View showing the sectional configuration of a portion of a gas sensor according to Embodiment 4.
[FIG. 8] Sectional view showing, on an enlarged scale, a sloping surface and its vicinity of a stacked body according to Embodiment 5.
[FIG. 9] Sectional view showing, on an enlarged scale, a sloping surface and its vicinity of a stacked body according to Embodiment 6.
[FIG. 10] Sectional view showing, on an enlarged scale, a sloping surface and its vicinity of a stacked body according to Embodiment 7.
[FIG. 11] Sectional view showing, on an enlarged scale, a sloping surface and its vicinity of a stacked body according to Embodiment 8.

### DESCRIPTION OF EMBODIMENTS

First, embodiments of the present disclosure will be listed and described.
(1) A gas sensor of the present disclosure comprises a stacked body including electrode layers and an insulating layer alternatingly stacked, wherein the stacked body has a sloping surface at a portion at least of its side surface, and wherein the sloping surface functions as a detection portion which detects a particular component of a gas under measurement.
(2) In the gas sensor described in (1), at least one of an uppermost layer and a lowermost layer of the stacked body may be a particular electrode layer having a horizontal surface which is continuous with the sloping surface and extends perpendicularly to a stacking direction, wherein a sensitive membrane is in contact with the side surface of the stacked body over the entirety of the side surface in the stacking direction, and wherein the sensitive membrane is in contact with a surface region extending from the horizontal surface to a portion of the sloping surface, the portion being continuous with the horizontal surface.
(3) In the gas sensor described in (2), both the uppermost layer and the lowermost layer of the stacked body may be particular electrode layers each having the horizontal surface, wherein the sensitive membrane is in contact with surface regions extending from the horizontal surfaces of the uppermost layer and the lowermost layer to portions of the sloping surface, the portions being continuous with the horizontal surfaces.
(4) In the gas sensor described in (2) or (3), the particular electrode layer may rise obliquely from its horizontal surface and constitute a portion of the sloping surface.
(5) In the gas sensor described in any of (2) to (4), the front surface of the stacked body has a horizontal surface extending perpendicularly to the stacking direction and the sloping surface which is continuous with the horizontal surface and is inclined in relation to the stacking direction, and an angle of the sloping surface at the electrode layer is preferably greater than an angle of the sloping surface at the insulating layer.

In the case where the gas sensor is configured as described, it becomes easier to dispose the sensitive membrane on the insulating layer.

(6) In the gas sensor described in any of (1) to (5), the front surface of the stacked body has a horizontal surface extending perpendicularly to the stacking direction and the sloping surface which is continuous with the horizontal surface and is inclined in relation to the stacking direction, and an angle of the sloping surface at the insulating layer is preferably greater than an angle of the sloping surface at the electrode layer.

In the case where the gas sensor is configured as described, it becomes possible to shorten the gap between the electrodes with the distance therebetween maintained constant.

(7) In the gas sensor described in any of (1) to (6), a heater which can heat the stacked body is provided in the insulating layer, and the heater is preferably disposed such that, when viewed in a stacking direction, the heater does not overlap with the sloping surface.

In the case where the gas sensor is configured as described, it becomes possible to suppress formation of a crack in the sensitive membrane by an edge portion of the heater.

### <Embodiment 1>

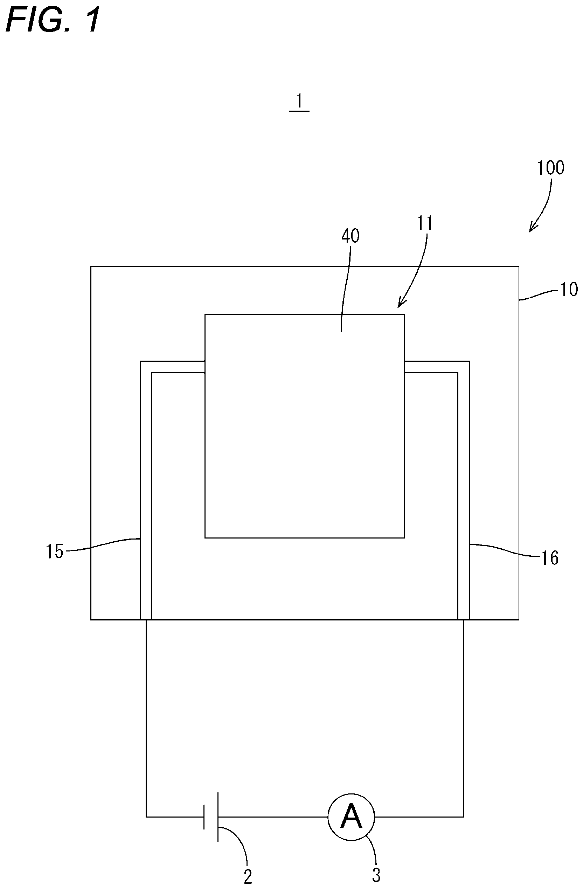

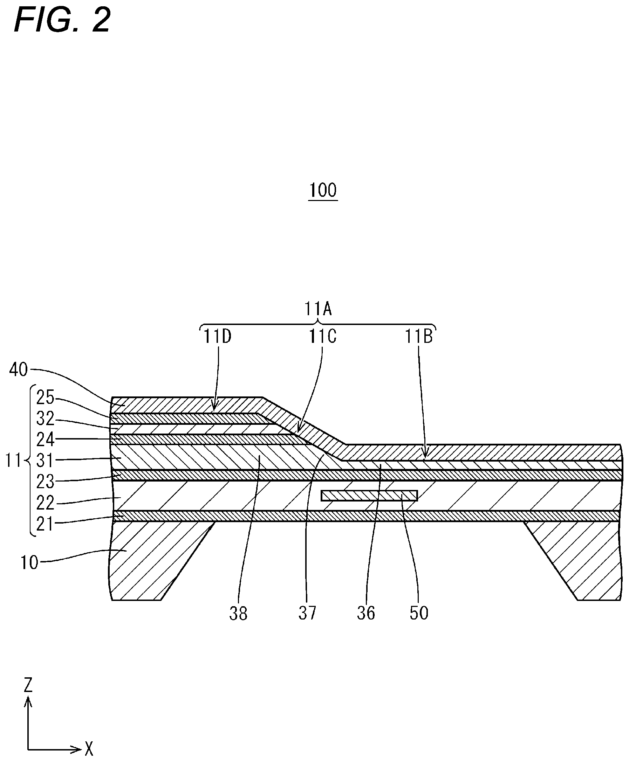

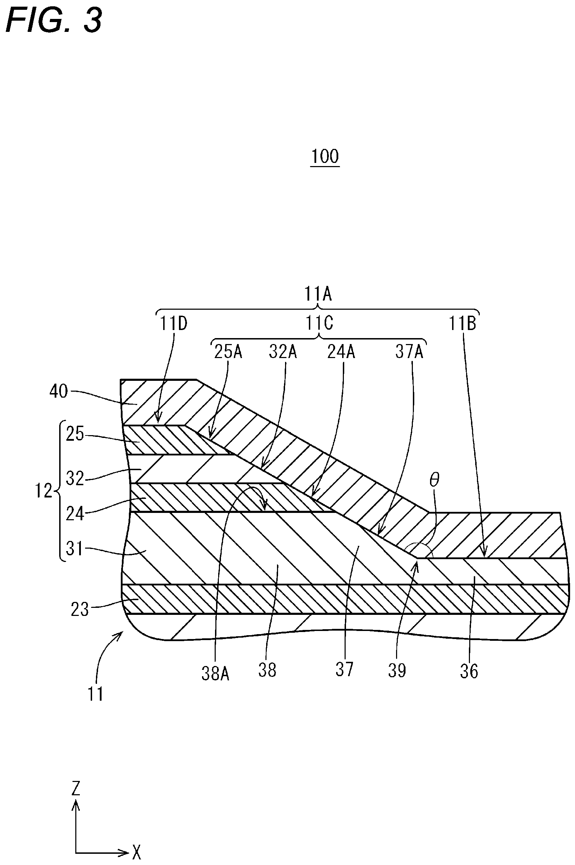

Embodiment 1 of the present disclosure will be described with reference to FIG. 1, etc. In the present embodiment, a gas sensor 100 which can detect a particular gas (gas under measurement) is shown as an example. Notably, in FIGS. 2 and 3, a Z direction corresponds to a stacking direction. and an X direction perpendicularly intersecting the Z direction corresponds to a horizontal direction. In addition, in FIG. 1, the near side of the sheet corresponds to the front surface side in the gas sensor 100 (the upper side in the stacking direction), and, in each of FIGS. 2 and 3, the upper side of the sheet corresponds to the front surface side in the gas sensor 100 (the upper side in the stacking direction). In addition, in FIG. 1, the far side of the sheet corresponds to the back surface side in the gas sensor 100 (the lower side in the stacking direction), and, in each of FIGS. 2 and 3, the lower side of the sheet corresponds to the back surface side in the gas sensor 100 (the lower side in the stacking direction).

As shown in FIG. 1, the gas sensor 100 includes a substrate 10 having a flat-platelike shape, a stacked body 11 stacked on the front surface of the substrate 10, and first and second wiring conductors 15 and 16 disposed on the front surface of the substrate 10 and connected to the stacked body 11. The substrate 10 is made of, for example, silicon. The gas sensor 100 constitutes a gas sensor device 1 together with a power supply 2 and an ammeter 3. The power supply 2 is connected to the first wiring conductor 15 and the ammeter 3. The ammeter 3 is connected to the second wiring conductor 16.

As shown in FIG. 2, the stacked body 11 includes a plurality of electrode layers 31 and 32 and a plurality of insulating layers 21, 22, 23, 24, and 25 which are stacked in the stacking direction (vertical direction). Specifically, the first insulating layer 21, the second insulating layer 22, and the third insulating layer 23 are stacked in this order on the front surface of the substrate 10. The first electrode layer 31, the fourth insulating layer 24, the second electrode layer 32, and the fifth insulating layer 25 are stacked in this order on the front surface of the third insulating layer 23. As to the layers from the third insulating layer 23 to the fifth insulating layer 25, the plurality of insulating layers 23, 24, and 25 and the plurality of electrode layers 31 and 32 are alternatingly stacked in the stacking direction. Notably, as shown in FIG. 3, the first electrode layer 31, the fourth insulating layer 24, the second electrode layer 32, and the fifth insulating layer 25 will be collectively referred to as a main body layer portion 12.

In the stacking direction, the distance between the first electrode layer 31 and the second electrode layer 32 (the thickness of the fourth insulating layer 24) is on the order of nanometer. The distance may be, for example, 1 nm or greater and less than 1000 nm, may be less than 100 nm, or may be several tens nm. The first wiring conductor 15 shown in FIG. 1 is connected to the first electrode layer 31. The second wiring conductor 16 is connected to the second electrode layer 32.

No particular limitation is imposed on the materials of the electrode layers 31 and 32 and the wiring conductors 15 and 16. Any material having electrical conductivity can be used. For example, of metals such as aluminum, copper, gold, platinum, etc., one or two or more metals can be employed. No particular limitation is imposed on the materials of the insulating layers 21, 22, 23, 24, and 25. Any material which does not have electrical conductivity can be used. Compounds such as SiO₂, Si₃N₄, etc. can be employed. A heater 50 which can heat the stacked body 11 is provided in the second insulating layer 22.

As shown in FIG. 2, the front surface 11A of the stacked body 11 has a first horizontal surface 11B which is a surface extending perpendicularly to the stacking direction (in the horizontal direction), a second horizontal surface 11D which is a surface extending perpendicularly to the stacking direction and located on the upper side in relation to the first horizontal surface 11B, and a sloping surface 11C which is a surface continuous with the first horizontal surface 11B and with the second horizontal surface 11D and inclined in relation to the stacking direction.

The first electrode layer 31 has a thin layer portion 36, a thick layer portion 38 thicker than the thin layer portion 36, and a gradually changing layer portion 37 which is located between the thin layer portion 36 and the thick layer portion 38 and whose thickness changes gradually in the horizontal direction. The thick layer portion 38 is thicker than the second electrode layers 32. As shown in FIG. 3, the fourth insulating layer 24 is stacked on the front surface (upper surface) 38A of the thick layer portion 38.

The first horizontal surface 11B is the front surface (upper surface) of the thin layer portion 36. The sloping surface 11C is formed by a continuous series of side surfaces; i.e., a side surface 37A of the gradually changing layer portion 37 (a side surface of the first electrode layer 31), a side surface 24A of the fourth insulating layer 24, a side surface 32A of the second electrode layer 32, and a side surface 25A of the fifth insulating layer 25. In the stacked body 11, the sloping surface 11C is the side surface of the main body layer portion 12. The second horizontal surface 11D is the front surface (upper surface) of the fifth insulating layer 25. The sloping surface 11C functions as a detection portion which can detect a particular gas (a particular component of a gas under measurement such as air).

The first electrode layer 31 is the lowest layer in the main body layer portion 12 and is a particular electrode layer which has the first horizontal surface 11B continuous with the sloping surface 11C. The first electrode layer 31, which is the particular electrode layer, has the side surface 37A which is the side surface 37A of the gradually changing layer portion 37, extends obliquely upward from the first horizontal surface 11B of the first electrode layer 31, and constitutes a portion of the sloping surface 11C. The angle θ of a corner portion 39 formed between the first horizontal surface 11B and the sloping surface 11C (or the side surface 37A) is an obtuse angle greater than 90 degrees. Specifically, the angle θ is preferably set to fall within the range of 120 degrees to 150 degrees, inclusive, and is particularly preferably set to fall within the range of 130 degrees to 140 degrees, inclusive.

The gas sensor 100 includes a sensitive membrane 40 which extends over and is in contact with the entirety (in the stacking direction) of the side surface (the sloping surface 11C) of the stacked body 11. In addition, the sensitive membrane 40 is in contact with a surface region extending from the first horizontal surface 11B to the side surface 37A of the gradually changing layer portion 37 (a portion of the sloping surface 11C which is continuous with the first horizontal surface 11B). The sensitive membrane 40 is in contact with the corner portion 39. Moreover, the sensitive membrane 40 is in contact with a surface region extending from the sloping surface 11C to the second horizontal surface 11D.

The sensitive membrane 40 is configured such that it reacts with a particular gas and its electrical characteristic (for example, resistance) changes accordingly. No particular limitation is imposed on the material of the sensitive membrane 40 so long as the material is appropriately selected in accordance with a gas to be detected. For example, an oxide semiconductor film can be employed. The oxide semiconductor film may be formed of any of ZnO, SnO₂, WO₃, In₂O₃, TiO₂, V₂O₅, etc. When, in the gas sensor 100, the particular gas reacts with the sensitive membrane 40 and the resistance of the sensitive membrane 40 changes, in the gas sensor device 1, the current value detected by the ammeter 3 connected to the power supply 2 changes. Thus, the gas sensor device 1 can detect the particular gas.

Notably, no particular limitation is imposed on a method of manufacturing the gas sensor 100. For example, in a step of forming the stacked body 11, the sloping surface 11C and the first horizontal surface 11B may be formed by performing ion etching from the front surface side of the fifth insulating layer 25.

Next, the effect of the present embodiment will be described. The gas sensor 100 of the present embodiment includes the stacked body 11 in which the electrode layers 31 and 32 and the insulating layer 24 are alternatingly stacked. The stacked body 11 has the sloping surface 11C at a portion at least of the side surface, and the sloping surface 11C functions as a detection portion which detects a particular component of the gas under measurement.

By virtue of such a configuration, it is possible to improve the sensitivity of detection by increasing the area of a region where the particular component of the gas under measurement can be detected, as compared with the case where the portion functioning as a detection portion extends vertically on the side surface of the stacked body 11.

At least one of the uppermost layer and the lowermost layer of the stacked body 11 is the particular electrode layer 31 having the horizontal surface 11B which is continuous with the sloping surface 11C and extends perpendicularly to the stacking direction, and the sensitive membrane 40 is in contact with the entirety (in the stacking direction) of the side surface of the stacked body 11. The sensitive membrane 40 is in contact with a surface region extending from the horizontal surface 11B to a portion of the sloping surface 11C, the portion being continuous with the horizontal surface 11B.

According to such a configuration, the gas sensor 100 includes the sensitive membrane 40 which is in contact with the entirety (in the stacking direction) of the side surface of the stacked body 11 in which the electrode layers 31 and 32 and the insulating layer 24 are alternatingly stacked. Thus, the gas under measurement reacts with the sensitive membrane 40 and the electrical characteristic (for example, resistance) of the sensitive membrane 40 changes, whereby detection of the gas under measurement through the electrode layers 31 and 32 can be performed with high sensitivity and high selectivity. In addition, the stacked body 11 has the sloping surface 11C disposed on at a portion at least of the side surface of the stacked body 11 and the particular electrode layer 31 having the horizontal surface 11B, and the sensitive membrane 40 is in contact with a surface region extending from the horizontal surface 11B to a portion of the sloping surface 11C, the portion being continuous with the horizontal surface 11B. Therefore, as compared with the case where the side surface of the stacked body 11 extends vertically, the area of contact with the sensitive membrane 40 can be increased, and an anchor effect can be obtained. Thus, the detection sensitivity of the gas sensor 100 can be improved. For example, in the case where the horizonal surface and the side surface of the stacked body intersect perpendicularly to each other and the sensitive membrane is in contact with these surfaces or in the case where a plurality of electrode layers are disposed on the front surface of the insulating layer and the sensitive membrane is in contact with these electrode layers, since the sensitive membrane is formed such that it is bent (for example, at right angle) so as to follow the shapes of the electrode layers , etc., there is a risk of occurrence of cracking or rupturing (this is called step breakage) at this bent portion. In contrast, in the above-described gas sensor 100, since the sensitive membrane 40 is in contact with a surface region extending from the horizontal surface 11B to a portion of the sloping surface 11C, the portion being continuous with the horizontal surface 11B, it is possible to avoid at least formation of the sensitive membrane 40 in such a manner that it is bent at right angle. Thus, the gas sensor 100 can have a configuration in which the area of contact between each layer and the sensitive membrane 40 is increased, while the step breakage is suppressed.

The particular electrode layer 31 rises obliquely from its horizontal surface 11B and constitutes a portion of the sloping surface 11C.

The gas sensor 100 as described above has a configuration in which the particular electrode layer 31 rises obliquely from its horizontal surface 11B and constitutes a portion of the sloping surface 11C, and the sensitive membrane 40 is in contact with a surface region extending from the horizontal surface 11B to a portion of the sloping surface 11C, the portion being continuous with the horizontal surface 11B. Therefore, it is possible to further increase the area of contact with the sensitive membrane 40, thereby improving the detection sensitivity. In addition, in one electrode layer 31 (the particular electrode layer 31), the sensitive membrane 40 is in contact with its horizontal surface 11B and sloping surface. Thus, the gas sensor 100 can have a configuration in which the sensitive membrane 40 is in sufficient contact with the electrode layer 31.

### <Embodiment 2>

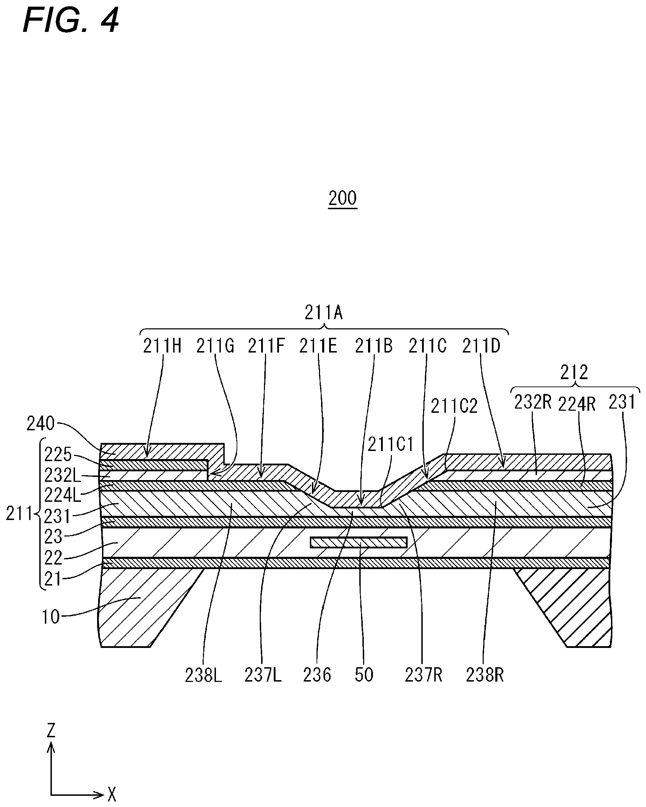

Next, Embodiment 2 of the present disclosure will be described with reference to FIG. 4. In the present embodiment, portions identical to those of the above-described embodiment are denoted by the same signs, and, as to its structure, action, and effect, their redundant descriptions are omitted.

In a gas sensor 200, a stacked body 211 includes a plurality of electrode layers 231, 232R, and 232L and a plurality of insulating layers 21, 22, 23, 224R, 224L, and 225 which are stacked in the stacking direction (vertical direction). The first electrode layer 231, the fourth insulating layers 224R and 224L, the second electrode layers 232R, and 232L, and the fifth insulating layer 225 are stacked in this order on the front surface of the third insulating layer 23. As to the layers from the third insulating layer 23 to the fifth insulating layer 225, the plurality of insulating layers 23, 224R, 224L, and 225 and the plurality of electrode layers 231, 232R, and 232L are alternatingly stacked in the stacking direction. The second electrode layers 232R and 232L and the fourth insulating layers 224R and 224L include layers (the second electrode layer 232R and the fourth insulating layer 224R) disposed on the right side of the sheet in relation to a first horizontal surface 211B, which will be described later, and layers (the second electrode layer 232L and the fourth insulating layer 224L) disposed on the left side of the sheet in relation to the first horizontal surface 211B. Notably, the first electrode layer 231, the fourth insulating layer 224R on the right side, and the second electrode layer 232R on the right side will be collectively referred to as a main body layer portion 212.

The front surface 211A of the stacked body 211 has a first horizontal surface 211B which is a surface extending horizontally, a second horizontal surface 211D which is a surface extending horizontally and is located on the upper side in relation to the first horizontal surface 211B, and a first sloping surface (sloping surface) 211C which is a surface continuous with the first horizontal surface 211B and the second horizontal surface 211D and inclined in relation to the stacking direction. In addition, the front surface 211A has a third horizontal surface 211F which is a surface extending horizontally and is located on the upper side in relation to the first horizontal surface 211B and on the lower side in relation to the second horizontal surface 211D, a second sloping surface (sloping surface) 211E which is a surface continuous with the first horizontal surface 211B and the third horizonal surface 211F and inclined in relation to the stacking direction, a fourth horizontal surface 211H which is a surface extending horizontally located on the upper side in relation to the second horizontal surface 211D, and an end surface 211G which is continuous with the third horizontal surface 211F and the fourth horizonal surface 211H and extends along the stacking direction,

The first electrode layer 231 has a thin layer portion 236, thick layer portions 238R and 238L thicker than the thin layer portion 236, and gradually changing layer portions 237R and 237L which are located between the thin layer portion 236 and the thick layer portions 238R and 238L and whose thicknesses change gradually in the horizontal direction. The thick layer portions 238R and 238L are thicker than the second electrode layers 232R and 232L. The gradually changing layer portions 237R and 237L and the thick layer portions 238R and 238L include layers (237R and 238R) located on the right side of the sheet in relation to the thin layer portion 236 and layers (237L and 238L) located on the left side of the sheet in relation to the thin layer portion 236.

The first horizontal surface 211B is the front surface of the thin layer portion 236. The first sloping surface 211C is a portion of the side surface (the first sloping surface 211C, the second sloping surface 211E, and the end surface 211G) of the stacked body 211 and the side surface of the main body layer portion 212. The first sloping surface 211C is formed by a continuous series of side surfaces; i.e., a side surface of the right-side gradually changing layer portion 237R, a side surface of the right-side fourth insulating layer 224R, and a side surface of the right-side second electrode layer 232R. The second sloping surface 211E is formed by a continuous series of side surfaces; i.e., a side surface of the left-side gradually changing layer portion 237L and a side surface of the left-side fourth insulating layer 224L. The third horizontal surface 211F is the front surface (upper surface) of the left-side fourth insulating layer 224L. The end surface 211G is composed of an end surface of the left-side second electrode layer 232L and an end surface of the fifth insulating layer 225 which are continuous with each other. The fourth horizontal surface 211H is the front surface (upper surface) of the fifth insulating layer 225.

The lowermost layer (the first electrode layer 231) and the uppermost layer (the right-side second electrode layer 232R) in the main body layer portion 212 of the stacked body 211 are both particular electrode layers having the horizontal surfaces 211B and 211D. The first electrode layer 231 is the lowermost layer in the main body layer portion 212 and is a first particular electrode layer which has the first horizontal surface 211B continuous with the sloping surface 211C. Ther right-side second electrode layer 232R is the uppermost layer in the main body layer portion 212 and is a second particular electrode layer which has the second horizontal surface 211D continuous with the sloping surface 211C. A sensitive membrane 240 is in contact with surface regions extending from the horizontal surfaces 211B and 211D of the lowermost layer (the first electrode layer 231) and the uppermost layer (the right-side second electrode layer 232R) to portions (sloping end portions 211C1 and 211C2) of the slopping surface 211C, the portions being continuous with the horizontal surfaces 211B and 211D. By virtue of such a configuration, it is possible to further increase the area of contact between the stacked body 211 and the sensitive membrane 240, thereby improving the detection sensitivity.

Notably, the sensitive membrane 240 is in contact with the entirety of the front surface 211A of the stacked body 211. Specifically, the sensitive membrane 240 is in contact with the first horizontal surface 211B, the first sloping surface 211C, the second horizontal surface 211D, the second sloping surface 211E, the third horizonal surface 211F, the end surface 211G, and the fourth horizontal surface 211H.

### <Embodiment 3>

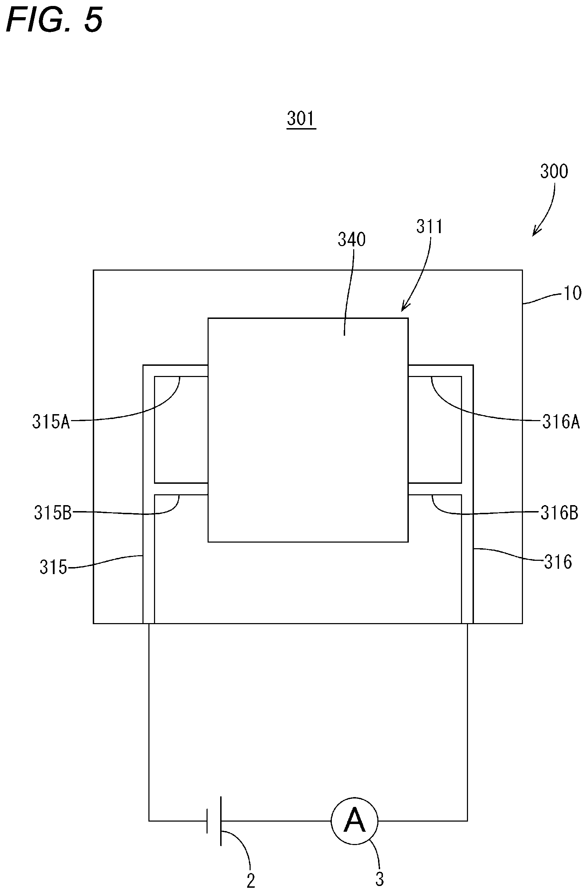

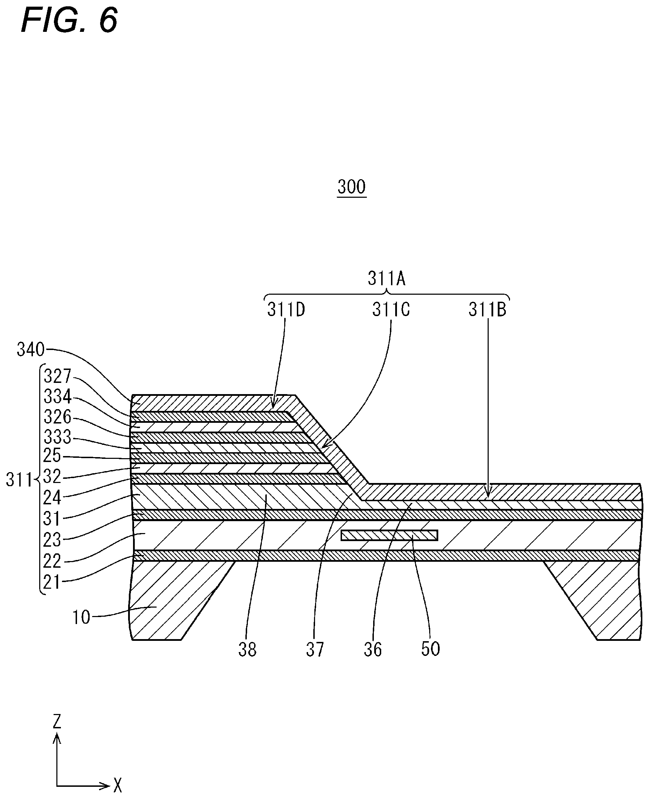

Next, Embodiment 3 of the present disclosure will be described with reference to FIGS. 5 and 6. In the present embodiment, portions identical to those of the above-described embodiment are denoted by the same signs, and, as to its structure, action, and effect, their redundant descriptions are omitted.

As shown in FIG. 5, a gas sensor 300 includes a first wiring conductor 315 and a second wiring conductor 316 connected to a stacked body 311. The first wiring conductor 315 is branched into a far-side wiring conductor 315A and a near-side wiring conductor 315B. Similarly, the second wiring conductor 316 is branched into a far-side wiring conductor 316A and a near-side wiring conductor 316B. The gas sensor 300 constitutes a gas sensor device 301 together with the power supply 2 and the ammeter 3.

As shown in FIG. 6, the stacked body 311 includes a plurality of electrode layers 31, 32, 333, and 334 and a plurality of insulating layers 21, 22, 23, 24, 25, 326, and 327, which are stacked in the stacking direction (vertical direction). The third electrode layer 333, the sixth insulating layer 326, the fourth electrode layer 334, and the seventh insulating layer 327 are stacked in this order on the front surface of the fifth insulating layer 25. The far-side wiring conductor 315A of the first wiring conductor 315 shown in FIG. 1 is connected to the first electrode layer 31. The far-side wiring conductor 316A of the second wiring conductor 316 is connected to the second electrode layer 32. The near-side wiring conductor 315B of the first wiring conductor 315 is connected to the third electrode layer 333. The near-side wiring conductor 316B of the second wiring conductor 316 is connected to the fourth electrode layer 334.

On the front surface 311A of the stacked body 311, the first horizontal surface 311B is the front surface (upper surface) of the thin layer portion 36. A sloping surface 311C is formed by a continuous series of side surfaces; i.e., a side surface of the gradually changing layer portion 37, a side surface of the fourth insulating layer 24, a side surface of the second electrode layer 32, a side surface of the fifth insulating layer 25, a side surface of the third electrode layer 333, a side surface of the sixth insulating layer 326, a side surface of the fourth electrode layer 334, and a side surface of the seventh insulating layer 327. The second horizontal surface 311D is the front surface (upper surface) of the seventh insulating layer 327. A sensitive membrane 340 is in contact with a surface region extending from the first horizontal surface 311B to the sloping surface 311C and then to the second horizontal surface 311D.

### <Embodiment 4>

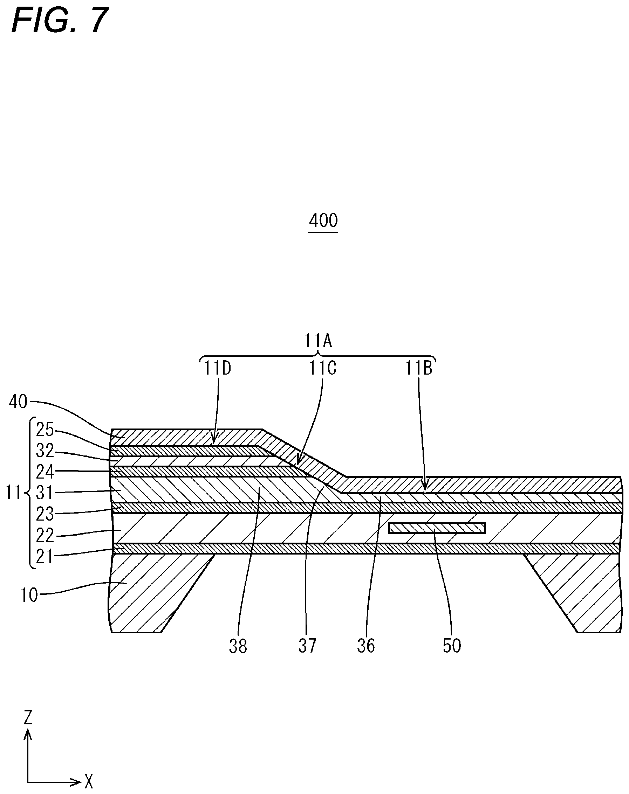

Next, Embodiment 4 of the present disclosure will be described with reference to FIG. 7. The present embodiment is one that is obtained by changing the position of the heater 50 of Embodiment 1. In the present embodiment, portions identical to those of the above-described Embodiment 1 are denoted by the same signs, and, as to its structure, action, and effect, their redundant descriptions are omitted.

As shown in FIG. 7, a stacked body 11 of a gas sensor 400 includes a plurality of electrode layers 31 and 32 and a plurality of insulating layers 21, 22, 23, 24, and 25 which are stacked in the stacking direction (vertical direction). A heater 50 which can heat the stacked body 11 is provided in the second insulating layer 22.

The front surface 11A of the stacked body 11 has a first horizontal surface 11B which is a surface extending perpendicularly to the stacking direction (in the horizontal direction), a second horizontal surface 11D which is a surface extending perpendicularly to the stacking direction and located on the upper side in relation to the first horizontal surface 11B, and a sloping surface 11C which is a surface continuous with the first horizontal surface 11B and with the second horizontal surface 11D and inclined in relation to the stacking direction. The heater 50 is disposed such that, when viewed in the stacking direction, the heater 50 does not overlap with the sloping surface 11C and overlaps with the first horizontal surface 11B. In the case where the heater is disposed such that it overlaps with the sloping surface, there is a possibility that a clack is formed in the sensitive membrane 40 and the sensitive membrane 40 becomes unable to function well. Therefore, in the present embodiment, the heater 50 is disposed such that the heater 50 does not overlap with the sloping surface 11C.

### <Embodiment 5>

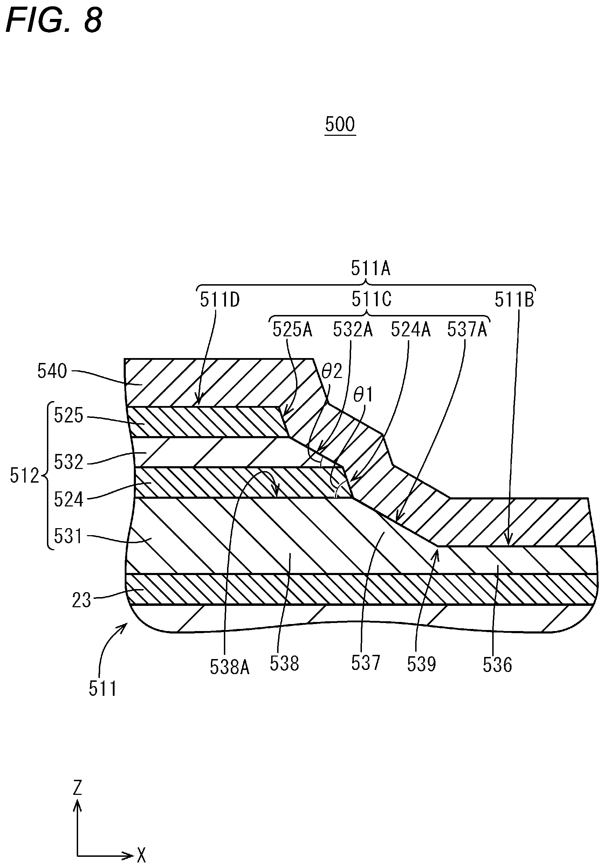

Next, Embodiment 5 of the present disclosure will be described with reference to FIG. 8. The present embodiment is one that is obtained by changing the shape of the sloping surface 11C of Embodiment 1. In the present embodiment, portions identical to those of the above-described Embodiment 1 are denoted by the same signs, and, as to its structure, action, and effect, their redundant descriptions are omitted.

As shown in FIG. 8, a stacked body 511 of a gas sensor 500 includes a plurality of electrode layers 531 and 532 and a plurality of insulating layers 23, 524, and 525 which are stacked in the stacking direction (vertical direction). The first electrode layer 531, the fourth insulating layer 524, the second electrode layer 532, and the fifth insulating layer 525 will be collectively referred to as a main body layer portion 512.

The front surface 511A of the stacked body 511 has a first horizontal surface 511B which is a surface extending perpendicularly to the stacking direction (in the horizontal direction), a second horizontal surface 511D which is a surface extending perpendicularly to the stacking direction and located on the upper side in relation to the first horizontal surface 511B, and a sloping surface 511C which is a surface continuous with the first horizontal surface 511B and with the second horizontal surface 511D and inclined in relation to the stacking direction.

The sloping surface 511C is formed by a continuous series of side surfaces; i.e., a side surface 537A of the gradually changing layer portions 537 (a side surface of the first electrode layer 531), a side surface 524A of the fourth insulating layer 524, a side surface 532A of the second electrode layer 532, and a side surface 525A of the fifth insulating layer 525. In the stacked body 511, the sloping surface 511C is the side surface of the main body layer portion 512.

The upper surface 538A of the first electrode layer 531 is parallel to the first horizontal surface 511B. The angle of the side surface 524A of the fourth insulating layer 524 which forms an acute angle in relation to the first horizontal surface 511B is equal to the angle θ1 of the side surface 524A of the fourth insulating layer 524 which forms an acute angle in relation to the upper surface 538A of the first electrode layer 531. The angle θ1 is the angle of the sloping surface 511C in the fourth insulating layer 524.

The upper surface of the fourth insulating layer 524 is parallel to the first horizontal surface 511B. The angle of the side surface 532A of the second electrode layer 532 which forms an acute angle in relation to the first horizontal surface 511B is equal to the angle θ2 of the side surface 532A of the second electrode layer 532 which forms an acute angle in relation to the upper surface of the fourth insulating layer 524. The angle θ2 is the angle of the sloping surface 511C in the second electrode layer 532.

In the present embodiment, the angle θ1 is larger than the angle θ2, and therefore, it becomes easier to dispose the sensitive membrane 540 on the side surface 532A of the second electrode layer 532.

### <Embodiment 6>

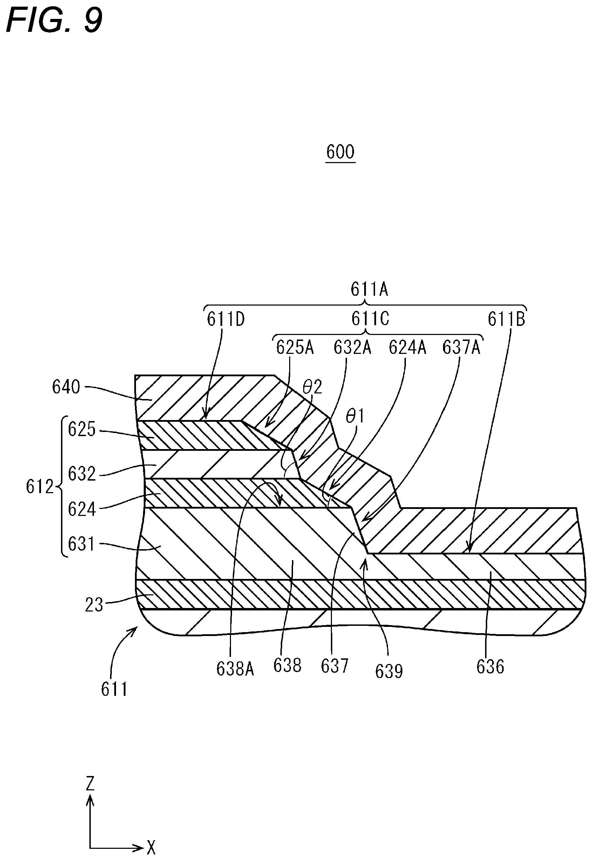

Next, Embodiment 6 of the present disclosure will be described with reference to FIG. 9. The present embodiment is one that is obtained by changing the shape of the sloping surface 511C of Embodiment 5. Portions identical to those of Embodiment 5 are denoted by the same signs, and, as to its structure, action, and effect, their redundant descriptions are omitted. Constituent elements corresponding to those of Embodiment 5 are denoted by signs each obtained by changing the number at the hundreds place of the numeral portion of a corresponding sign from 5 to 6.

In the present embodiment, since the angle θ2 is larger than the angle θ1, it is possible to shorten the gap between a first electrode layer 631 and a second electrode layer 632 with the distance therebetween maintained constant.

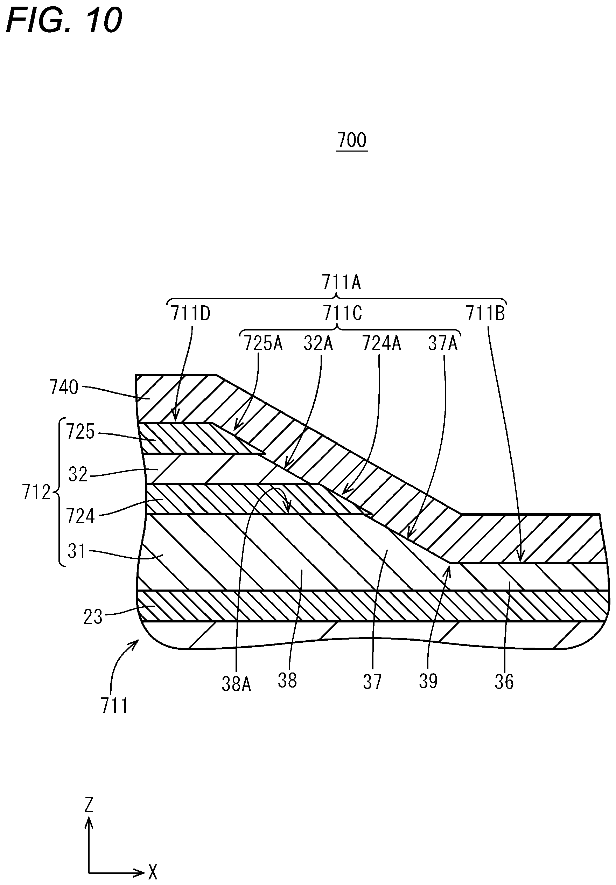

Next, Embodiment 7 of the present disclosure will be described with reference to FIG. 10. The present embodiment is one that is obtained by modifying Embodiment 1 such that the sloping surface 11C has positional shifts. Portions identical to those of Embodiment 1 are denoted by the same signs, and, as to its structure, action, and effect, their redundant descriptions are omitted. Constituent elements corresponding to those of Embodiment 1 are denoted by signs each obtained by adding 7 at the hundreds place of the numeral portion of a corresponding sign.

In the present embodiment, a side surface 724A of a fourth insulating layer 724 and a side surface 725A of a fifth insulating layer 725 protrude outward in relation to the side surface 37A of the gradually changing layer portion 37 and the side surface 32A of the second electrode layer 32.

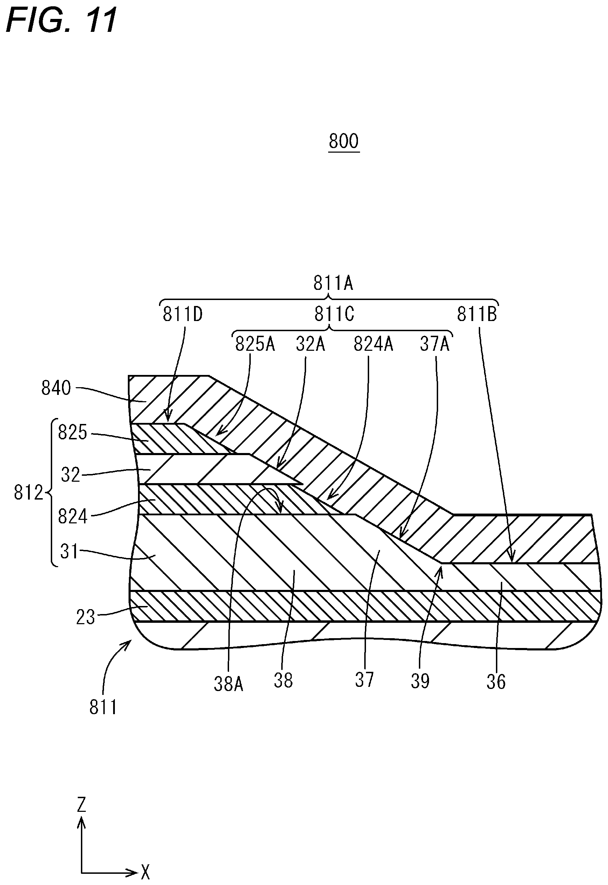

Next, Embodiment 8 of the present disclosure will be described with reference to FIG. 11. The present embodiment is one that is obtained by modifying Embodiment 1 such that the sloping surface 11C has positional shifts. Portions identical to those of Embodiment 1 are denoted by the same signs, and, as to its structure, action, and effect, their redundant descriptions are omitted. Constituent elements corresponding to those of Embodiment 1 are denoted by signs each obtained by adding 8 at the hundreds place of the numeral portion of a corresponding sign.

In the present embodiment, a side surface 824A of a fourth insulating layer 824 and a side surface 825A of a fifth insulating layer 825 are recessed in relation to the side surface 37A of the gradually changing layer portion 37 and the side surface 32A of the second electrode layer 32.

### <Other embodiments>

The present disclosure is not limited to the embodiments described by the above description and the drawings, and, for example, the following embodiments fall within the technical scope of the present disclosure. Moreover, in addition to the following modifications, various modifications may be employed for implementation of the technique of the present disclosure without departing from the gist.
(1) The configuration of the stacked body can be changed appropriately from the configurations employed in the above-described embodiments. It is sufficient that the stacked body includes at least a first electrode layer, an insulating layer stacked on the first electrode layer, and a second electrode layer stacked on the insulating layer. In such a configuration, the upper surface of the second electrode layer may be in contact with a sensitive membrane.
(2) The configuration of the sensitive membrane can be changed appropriately from the configurations employed in the above-described embodiments. The sensitive membrane may be configured such that it is in contact with the first horizontal surface and the sloping surface and is not in contact with the second horizontal surface. Alternatively, the sensitive membrane may be configured such that it is in contact with a portion of the first horizontal surface and the sloping surface which is continuous with the portion of the first horizontal surface.
(3) The configuration of the wiring conductors can be changed appropriately. In the above-described embodiments, the wiring conductors are routed to be connected to the sides of the stacked body located on opposite sides in the lateral direction (the left side and right side of the sheet in FIG. 1). However, the routing is not limited thereto. For example, the wiring conductors may be routed to be connected to the side of the stacked body located on the near side (the lower side of the sheet in FIG. 1).
(4) The instrument used for measuring the electrical characteristics in the gas sensor device is not limited to an ammeter for measuring current, and measuring instruments for measuring various electrical characteristics such as a voltmeter and an ohmmeter may be employed.

### REFERENCE SIGNS LIST

10: substrate, 11, 211, 311, 511, 611, 711, 811: stacked body, 11B, 211B, 311B, 511B, 611B, 711B, 811B : first horizontal surface (horizontal surface), 11C, 211C, 311C, 511C, 611C, 711C, 811C: sloping surface, 24, 224R, 524, 624, 724, 824: fourth insulating layer (insulating layer), 31, 32, 231, 232R, 531, 631: electrode layer, 40, 240, 340, 440, 540, 640, 740, 840: sensitive membrane, 100, 200, 300, 400, 500, 600, 700, 800: gas sensor

## Claims

1. A gas sensor comprising a stacked body including electrode layers and an insulating layer alternatingly stacked,
wherein the stacked body has a sloping surface at a portion at least of its side surface, and
wherein the sloping surface functions as a detection portion which detects a particular component of a gas under measurement.

2. The gas sensor according to claim 1, wherein at least one of an uppermost layer and a lowermost layer of the stacked body is a particular electrode layer having a horizontal surface which is continuous with the sloping surface and extends perpendicularly to a stacking direction,
wherein a sensitive membrane is in contact with the entirety in the stacking direction of the side surface of the stacked body, and
wherein the sensitive membrane is in contact with a surface region extending from the horizontal surface to a portion of the sloping surface, the portion being continuous with the horizontal surface.

3. The gas sensor according to claim 2, wherein both the uppermost layer and the lowermost layer of the stacked body are particular electrode layers each having the horizontal surface, and
wherein the sensitive membrane is in contact with surface regions extending from the horizontal surfaces of the uppermost layer and the lowermost layer to portions of the sloping surface, the portions being continuous with the horizontal surfaces.

4. The gas sensor according to claim 2 or 3, wherein the particular electrode layer rises obliquely from its horizontal surface and constitutes a portion of the sloping surface.

5. The gas sensor according to claim 2, wherein a front surface of the stacked body has a horizontal surface extending perpendicularly to the stacking direction and the sloping surface which is continuous with the horizontal surface and is inclined in relation to the stacking direction, and
wherein an angle of the sloping surface at the electrode layer is greater than an angle of the sloping surface at the insulating layer.

6. The gas sensor according to claim 1, wherein a front surface of the stacked body has a horizontal surface extending perpendicularly to the stacking direction and the sloping surface which is continuous with the horizontal surface and is inclined in relation to the stacking direction, and
wherein an angle of the sloping surface at the insulating layer is greater than an angle of the sloping surface at the electrode layer.

7. The gas sensor according to claim 1, wherein a heater which can heat the stacked body is provided in the insulating layer, and
wherein the heater is disposed such that, when viewed in a stacking direction, the heater does not overlap with the sloping surface.
